# EUROPÄISCHE PATENTSCHRIFT

(11) **EP 0 983 982 B1**
(45) Veröffentlichungstag und Bekanntmachung des Hinweises auf die Patenterteilung: **05.06.2002**
(21) Anmeldenummer: 99116177.9
(22) Anmeldetag: 23.08.1999
(51) Int. Cl.: C07B 45/00, C07C 303/02, C07C 309/86, C07D 213/71

(54) **Verfahren zur Herstellung von aromatischen oder heteroaromatischen Sulfonsäurechloriden**
Process for the preparation of aromatic or heteroaromatic sulfonyl chlorides
Procédé pour la préparation de chlorures de sulfonyle aromatiques ou hétéroaromatiques

(30) Priorität: 04.09.1998 DE 19840319
(43) Veröffentlichungstag der Anmeldung: 08.03.2000
(73) Patentinhaber: Bayer Aktiengesellschaft, 51368 Leverkusen (DE)
(72) Erfinder: Schnatterer, Albert, Dr., 51373 Leverkusen (DE); Hermann, Monika, 51503 Rösrath (DE)

(56) Entgegenhaltungen:
- EP-A- 0 059 241
- DE-A- 2 308 262

## Beschreibung

Die vorliegende Erfindung betrifft ein Verfahren zur Herstellung von aromatischen oder heteroaromatischen Sulfonsäurechloriden durch Diazotierung von Amino-substituierten Aromaten oder Heteroaromaten und Zersetzung der erhaltenen Diazoniumsalze in Gegenwart von Schwefeldioxid.

Aromatische Sulfonsäurechloride sind wichtige Zwischenprodukte für Pflanzenschutz-produkte, Pharmazeutika und Farbstoffe. Aus Chem. Ber. 90, 1957, S. 841 ist es bekannt, diese Verbindungsklasse durch Diazotierung der entsprechenden aromatischen Amine mit wässrigen Natriumnitritlösungen und Zersetzung der Diazoniumsalze in Lösungen von Schwefeldioxid, Kupfersalzen und Essigsäure herzustellen. Gegebenenfalls wird ferner ein mit Wasser nicht mischbares organisches Lösungsmittel zugegeben, so daß das Reaktionsgemisch zweiphasig ist. Dieses Verfahren hat jedoch eine Reihe von Nachteilen. So sind die Ausbeuten in vielen Fällen nicht befriedigend. Während sich elektronenanziehende Gruppen als weitere Substituenten in den Diazoniumsalzen günstig auf die Ausbeute und die Reaktionsgeschwindigkeit auswirken, führt der Einsatz von Diazoniumsalzen, die elektronenabgebende Substituenten tragen, nur zu geringen Ausbeuten. Ferner fallen bei einer großtechnischen Durchführung der Reaktion große Mengen an Kupfer und Essigsäure enthaltenden Abwässer an. Die Aufarbeitung und Entsorgung solcher Abwässer ist kostenintensiv und läßt sich nicht mit einem wirtschaftlichen Verfahren vereinbaren. Bei Einsatz von Diazoniumsalzen mit elektronenanziehenden Substituenten am aromatischen Ring, wie z.B. Dinitrobenzol-diazoniumchloriden, kann das Verfahren gegebenenfalls auch ohne die Kupfersalze als Katalysator durchgeführt werden. Während die Ausbeuten in diesem Fall nicht viel geringer sind als bei Anwesenheit des Katalysators, sinkt jedoch die Reaktionsgeschwindigkeit erheblich ab.

Aus der DE-OS 23 08 262 ist ein weiteres Verfahren zur Herstellung von aromatischen Sulfonsäurechloriden bekannt, bei dem das Schwefeldioxid in Form von Alkalihydrogensulfit in Gegenwart von Kupfer oder Kupfersalzen eingesetzt wird. Der Zusatz von Essigsäure oder anderen organischen Lösungsmitteln ist hierbei nicht erforderlich, die aromatischen Diazoniumsalze werden in stark salzsaurer Lösung verwendet. Nach EP-A-0 059 241 wird die Umsetzung der wässrigen Diazoniumsalzlösung mit Schwefeldioxid in Gegenwart eines mit Wasser nicht oder nur begrenzt mischbaren Lösungsmittels sowie Kupfersalzen als Katalysator durchgeführt und im Anschluß daran das Reaktionsgemisch mit einem Oxidationsmittel behandelt. Diese Verfahren vermeiden zwar das Problem des Anfalls von Essigsäure enthaltenden Abwässern, durch die Diazotierung der Aniline in wässrigem Medium und den Einsatz der verdünnten wässrigen Diazoniumsalzlösungen bei der Reaktion mit dem Schwefeldioxid in Gegenwart von Kupfer fallen jedoch wiederum große Mengen an Kupfersalze enthaltenden Abwässer an. Außerdem ist die Raum-Zeit-Ausbeute dieser Verfahren durch den Einsatz der verdünnten wässrigen Diazoniumsalzlösung und der dadurch bedingten großen Volumina unbefriedigend. Die Handhabung der Diazoniumsalzlösungen erfordert wegen der Zersetzbarkeit der Diazoniumsalze zudem besondere Vorsichtsmaßnahmen. So müssen diese Lösungen bei tiefen Temperaturen um 0°C gehandhabt und rasch verarbeitet werden, um im technischen Betrieb die Ausbeuteverluste zu begrenzen.

Allen zuvor genannten Verfahren gemeinsam ist der Nachteil, daß die Umwandlung der Aniline in die entsprechenden aromatischen Sulfonsäurechloride zweistufig erfolgt. Im ersten Schritt erfolgt die Diazotierung des Anilins mit wässriger Natriumnitritlösung und im zweiten Schritt die Zersetzung des Diazoniumsalzes in Gegenwart von Schwefeldioxid oder Alkalihydrogensulfit, einer Kupferverbindung und gegebenenfalls einem organischen Lösungsmittel.

Es bestand deshalb Bedarf an einem Verfahren, das die Herstellung von aromatischen oder heteroaromatischen Sulfonsäurechloriden aus den zugrundeliegenden Anilinen bzw. Amino-substituierten Pyridinen in einfacher Weise mit einer hohen chemischen Ausbeute, einer hohen Raum-Zeit-Ausbeute bei gleichzeitig geringem Anfall an Abwässern ermöglicht.

Gegenstand der Erfindung ist ein Verfahren zur Herstellung von aromatischen oder heteroaromatischen Sulfonsäurechloriden der allgemeinen Formel I 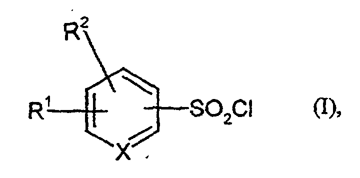 worin
- X: gleich C oder N,
- R¹: gleich Fluor, Chlor, Brom, Nitro, Methoxy, Fluormethoxy, Difluormethoxy, Trifluormethoxy, C₁ - C₄ Alkyl oder Phenyl, und
- R²: gleich H, Fluor, Chlor, Brom, OH oder C₁ - C₄ Alkyl ist,
durch Diazotierung der Verbindungen der allgemeinen Formel II 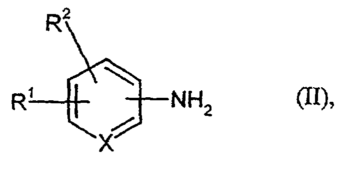 in der
- X, R¹ und R²: die für die Formel (I) angegebenen Bedeutungen haben,
und Zersetzung der daraus resultierenden Diazoniumsalze in Gegenwart von Schwefeldioxid und einem Kupferkatalysator,
dadurch gekennzeichnet, daß die Verbindungen der Formel (II) in einem Gemisch aus einem oder mehreren organischen Lösungsmitteln, Schwefeldioxid und einem Kupferkatalysator bei Temperaturen von -20 bis +60 °C mit Chlorwasserstoff und Alkylnitrit behandelt werden.

Soweit in den Formeln (I) und (II) R¹ bzw. R² für C₁-C₄ Alkyl steht, können diese Alkylreste geradkettig oder verzweigt sein. Beispielsweise können Methyl, Ethyl, n-Propyl, Isopropyl, n-Butyl, Isobutyl und tert.-Butyl eingesetzt werden.

In den Formeln (I) und (II) steht R¹ bevorzugt für Fluor, Chlor, Brom, Nitro, Methoxy, Trifluormethoxy, Methyl oder Ethyl und R² bevorzugt für Wasserstoff, Fluor, Chlor, Brom, Methyl oder Ethyl.

In den Formeln (I) und (II) steht R¹ besonders bevorzugt für Fluor, Chlor, Brom, Nitro oder Trifluormethoxy und R² besonders bevorzugt für Wasserstoff.

Wenn es sich bei den Verbindungen der Formel (II) um substituierte Aniline handelt, X somit für C steht, so befinden sich die Substituenten R¹ und R² bevorzugt in 2 und/oder 4-Stellung zum Aminorest. Wenn es sich bei den Verbindungen der Formel (II) um substituierte Amino-Pyridine handelt, X somit für N steht, so befindet sich der Aminorest bevorzugt in 3-Stellung und die Substituenten R¹ und R² bevorzugt in 2 und/oder 6-Stellung.

Einzelbeispiele für Verbindungen der Formel (II), auf die das erfindungsgemäße Verfahren mit Vorteil angewendet werden kann, sind 2-Nitroanilin, 4-Nitroanilin, 2-Trifluormethoxyanilin und 3-Amino-2-chlorpyridin. Diese Verbindungen sind käuflich erhältlich.

Als organische Lösungsmittel für das erfindungsgemäße Verfahren kommen solche in Frage, die unter den Reaktionsbedingungen im wesentlichen inert sind und die Edukte der Formel (I), Schwefeldioxid, Chlorwasserstoff und den Kupferkatalysator wenigstens teilweise lösen. Solche Lösungsmittel sind beispielsweise aromatische Kohlenwasserstoffe, Alkohole, Ether, halogenierte Kohlenwasserstoffe, Sulfoxide und Sulfone. Solche Lösungsmittel können einzeln oder als Gemische von zwei oder mehreren Lösungsmitteln eingesetzt werden.

Bevorzugt werden aromatische Kohlenwasserstoffe z. B. Toluol, Xylole, Ethylbenzol, halogenierte Kohlenwasserstoffe, wie z.B. Methylenchlorid, 1,2-Dichlorethan, Chlorbenzol oder Dichlorbenzol, Alkohole, wie z. B. Methanol, Ethanol, 1-Propanol, 2-Propanol, isomere Butanole, isomere Pentanole oder isomere Hexanole, oder Ether, wie z.B. Methyl-tert.-butylether, Tetrahydrofuran, Ethylenglykoldimethylether, Diethylenglykoldimethylether, Triethylenglykoldimethylether oder Polyethylen-glykoldimethylether als Lösungsmittel eingesetzt.

Solche Lösungsmittel können auch in Gemischen mit Wasser eingesetzt werden. Der Zusatz von Wasser kann in manchen Fällen die Löslichkeiten der Edukte begünstigen. Dabei ist es unerheblich, ob solche Gemische ein oder zwei flüssige Phasen bilden. Der Zusatz von Wasser ist aber verfahrensbedingt auf geringe Mengen von 0,1 bis 0,5 Gew.Teilen, bezogen auf die Gesamtmenge des organischen Lösungsmittels, beschränkt.

Die Menge des Lösungsmittels wird so bemessen, daß gut rührbare Lösungen oder Suspensionen erhalten werden, und beträgt je nach dessen Lösevermögen für die Ausgangskomponenten vorzugsweise 1 - 20 Gew. Teile, bevorzugt 1,5 - 10 Gew. Teile, bezogen auf die Verbindungen der Formel (II).

Zur Erzielung möglichst hoher Ausbeuten an Sulfonsäurechloriden der Formel (I) verwendet man zweckmäßigerweise mindestens äquimolare Mengen Schwefeldioxid bezogen auf die Verbindungen der Formel (II); es empfiehlt sich jedoch im allgemeinen, einen 1-8 molaren, bevorzugt 1 - 6 molaren, besonders bevorzugt 1,5 - 5 molaren Überschuß an Schwefeldioxid einzusetzen.

Das erfindungsgemäß einzusetzende Schwefeldioxid kann vor der Behandlung des Reaktionsgemisches mit Alkylnitrit dem Reaktionsgemisch vollständig zugesetzt werden. Es kann aber auch in Teilmengen vorgelegt werden und der Rest gleichzeitig mit dem Alkylnitrit zugegeben werden.

Als Kupferkatalysator kommen elementares Kupfer sowie Verbindungen des Kupfers in Betracht. Das Kupfer kann in dem einzusetzenden Kupferkatalysator die Wertigkeiten 0, 1, 2 oder 3 , bevorzugt 1 oder 2, annehmen. Mögliche Einsatzformen sind Kupfersalze anorganischer und organischer Säuren, beispielsweise Kupferfluorid, Kupferchlorid, Kupferbromid, Kupfersulfat, Kupfernitrat, ferner die Kupferoxide, die Kupfersalze organischer Säuren, wie Kupferacetat, Kupfercitrat und Kupferstearat, sowie Kupferkomplexe, wie Kupferacetylacetonat und N,N'-Disalicylidenethylendiamin-kupfer (II). Der Kupferkatalysator kann in Lösung, bevorzugt in alkoholischer und insbesondere in methanolischer Lösung eingesetzt werden.

Der Kupferkatalysator wird dabei in einer Menge von 0,001 - 0,5 Mol, bevorzugt 0.005 - 0.1 Mol, pro Mol der Verbindung der Formel (II) verwendet.

Chlorwasserstoff wird zweckmäßigerweise gasförmig eingesetzt. Der Einsatz kann simultan mit dem Alkylnitrit erfolgen. Es kann aber auch der gesamte Chlorwasserstoff oder eine Teilmenge zum Reaktionsgemisch dosiert werden, bevor diese mit Alkylnitrit behandelt wird. Die Chlorwasserstoffmenge wird wenigstens äquimolar bezogen auf das Anilin bzw. das Amino-substituierte Pyridin der Formel (II) bemessen. Bevorzugt kommt Chlorwasserstoff in einer Menge von 1 - 4 Äquivalenten, besonders bevorzugt 1-2 Äquivalenten, bezogen auf die Verbindung der Formel (II) zum Einsatz.

Als Alkylnitrit wird üblicherweise Methylnitrit, Ethylnitrit oder Isopropylnitrit eingesetzt. Bevorzugt wird mit Methylnitrit gearbeitet. Methylnitrit kann durch Umsetzung von Alkalinitriten mit Methanol in Gegenwart von starken Säuren in einfacher Weise hergestellt werden. Da es unter den Arbeitsbedingungen gasförmig ist, wird es als Gas in dem Maße dosiert, wie der Reaktionsverlauf es zuläßt.

Das Alkylnitrit wird dabei zur Erzielung hoher Ausbeuten wenigstens in äquivalenten Mengen, vorzugsweise in Mengen von 1-3 Äqivalenten, besonders bevorzugt 1-1,5 Äquivalenten, bezogen auf die Verbindungen der Formel (II), eingesetzt Auch ein darüber hinaus gehender Überschuß an Alkylnitrit beeinträchtigt jedoch das Ergebnis der Umsetzung nicht.

Die Reaktionstemperatur für das erfindungsgemäße Verfahren kann zwischen -20 und + 60 °C variieren. Bevorzugt wird bei einer Temperatur von -10 bis + 40 °C, besonders bevorzugt -10 bis +30 °C gearbeitet.

In einer bevorzugten Ausführungsform des erfindungsgemäßen Verfahrens werden zunächst die Verbindung der Formel (II), das organische Lösungsmittel und der Kupferkatalysator zusammengegeben, dann unter Einstellung einer Temperatur im Bereich von -20 bis +60°C ein Teil des Chlorwasserstoffs und das Schwefeldioxid zugegeben und dann weiterer Chlorwasserstoff sowie das Alkynitrit zudosiert.

Das erfindungsgemäße Verfahren zur Herstellung der aromatischen bzw. heteroaromatischen Sulfonsäurechloride unterscheidet sich von den bisher bekannten Verfahren dadurch, daß die Diazotierung der Aniline bzw. Amino-substituierten Pyridine und die Zersetzung der resultierenden Diazoniumsalze nicht in zwei getrennten Stufen, sondern gleichzeitig in einem einzigen Schritt erfolgt und daß die Reaktion ausschließlich in einem organischen Lösungsmittel oder einem Gemisch aus mehreren organischen Lösungsmitteln erfolgreich durchgeführt werden kann. Dadurch ergeben sich wesentliche Vorteile gegenüber den Verfahren des Standes der Technik: Durch die Gleichzeitigkeit von Diazotierung und Zersetzung des Diazoniumsalzes entfällt der Apparate- und Zeitaufwand für die separate Herstellung der Diazoniumsalzlösungen. Ferner wird eine besonders günstige Raum-Zeit-Ausbeute erzielt und Abwasser fällt nur in Form von Reaktionswasser an.

Es ist als besonders überraschend anzusehen und aus dem Stand der Technik nicht abzuleiten, daß die Diazotierung der Aniline bzw. Amino-substituierten Pyridine der Formel (II) und die gleichzeitige Umsetzung der entstehenden Diazoniumsalze zu Sulfonsäurechloriden sich in einem Gemisch aus Schwefeldioxid, Chlorwasserstoff, Kupferkatalysator und organischen Lösungsmitteln in hoher Ausbeute bewerkstelligen läßt. Aus dem Stand der Technik ist nämlich bekannt, daß das sich zersetzende Diazoniumsalz mit nicht umgesetztem Anilin zu unerwünschten Folgeprodukten reagieren könnte (Houben-Weyl, Methoden der organischen Chemie, 4. Auflage, Band X/3, Seite 700). Dieses Phänomen tritt jedoch im erfindungsgemäßen Verfahren nicht auf.

Die Isolierung der Sulfonsäurechloride aus den anfallenden Reaktionsgemischen gestaltet sich sehr einfach. Wurde die Umsetzung in einem organischen Lösungsmittel durchgeführt, das mit Wasser nicht unbegrenzt mischbar ist, so scheidet sich das Reaktionswasser zusammen mit dem als Katalysator eingesetzten Kupfersalz als eigene Phase ab. Gegebenenfalls führt man diese Phasentrennung durch Zusatz geringer Mengen Wasser herbei. Aus der verbleibenden organischen Phase wird anschließend der Überschuß an Schwefeldioxid und Chlorwasserstoff ausgetrieben. Das Sulfonsäurechlorid in der organischen Phase kann anschließend direkt weiterverarbeitet werden, beispielsweise zu Sulfonamiden durch Umsetzung mit Ammoniak oder Aminen.

### Beispiel 1

Das in den nachfolgenden Beispielen eingesetzte Methylnitrit wird nach Bedarf durch Behandeln einer Lösung aus 1 Gew. Teil. 25 %iger wässriger Natriumnitritlösung und 0,14 Gew. Teilen Methanol mit 0,37 Gew. Teilen 48 % iger Schwefelsäure hergestellt und gasförmig weiterverarbeitet

### Beispiel 2

In einen 1 l Glasreaktor werden nacheinander 88,6 g 2-Trifluormethoxyanilin (97,6 %), 250 ml Dichlorethan und eine Lösung von 2 g Kupfer(II)chlorid x 2 H₂O in 3 ml Methanol vorgelegt. In die resultierende Lösung werden bei einer Temperatur von 0-10 °C erst 18 g Chlorwasserstoff, dann 128 g Schwefeldioxid eingeleitet. Dabei bildet sich eine dünnflüssige Suspension aus. Unter Rühren werden anschließend bei 0-5 °C innerhalb von 1,5 Stunden gleichzeitig 9 g Chlorwasserstoff und 34 g Methylnitrit kontinuierlich eindosiert. Die Methylnitritdosierung wird von einer gleichmäßigen Gasentwicklung im Reaktionsgemisch begleitet. Gegen Ende der Methylnitritdosierung löst sich die Suspension vollständig auf. Die Lösung wird anschließend noch 2 Stunden bei 5-10 °C nachgerührt.

Das Reaktionsgemisch wird dann zur Abtrennung des Schwefeldioxid- und Chlorwasserstoff-Überschusses bei 20-25 °C und einem Druck von 75 mbar destilliert. Es verbleiben 248 g flüssiger Rückstand, der zur Abtrennung des Kupfersalzes mit 50 ml Wasser extrahiert wird. Nach Eindampfen der organische Phase bei 55 °C und einem Druck von 40 mbar fallen als Rückstand 128,9 g eines leicht rötlich gefärbten Öls mit einem über Gaschromatographie bestimmten Gehalt von 91,1 % 2-Trifluormethoxy-benzolsulfochlorid, entsprechend einer Ausbeute von 92,3 % der Theorie, an.

### Beispiel 3

In einen 1 l Glasreaktor werden nacheinander 88,6 g 2-Trifluormethoxyanilin (97,6 %), 250 ml 1.2-Dimethoxyethan und eine Lösung von 2 g Kupfer(II)chlorid x 2 H₂O in 3 ml Methanol vorgelegt. In die resultierende Lösung werden bei einer Temperatur von 0-10 °C erst 18 g Chlorwasserstoff und dann 128 g Schwefeldioxid eingeleitet. Nach vollständiger Schwefeldioxideinleitung liegt das Einsatzgemisch in Form einer klaren Lösung vor. Unter gutem Rühren werden anschließend bei 0-12°C innerhalb von 1,5 Stunden gleichzeitig 9 g Chlorwasserstoff und 34 g Methylnitrit kontinuierlich eindosiert. Die Methylnitritdosierung wird von einer gleichmäßigen Gasentwicklung im Reaktionsgemisch begleitet. Im Anschluß an die Methylnitritdosierung wird die Lösung noch 2 Stunden bei 5-10 °C nachgerührt. Das Reaktionsgemisch wird daraufhin bei 50 °C und 20 mbar eingedampft, der ölige Rückstand in 100 ml 1.2-Dichlorethan aufgenommen und mit 50 ml Wasser extrahiert. Nach dem Eindampfen der Dichlorethanphase bei 50 °C und 16 mbar fallen 124,8 g eines leicht rötlich gefärbten Öls mit einem über Gaschromatographie bestimmten Gehalt von 94,6 % 2-Trifluormethoxybenzolsulfochlorid als Rückstand an. Die Ausbeute beträgt 92,8 % der Theorie.

### Beispiel 4

In einen 1l Glasreaktor werden 250 ml Toluol, 20 ml Polyethylenglykol 400, 88,6 g 2-Trifluormethoxyanilin (97,6 %) und eine Lösung von 2 g Kupfer(II)chlorid x 2H₂O in 3 ml Methanol vorgelegt. In die resultierende Lösung werden anschließend bei 0-10 °C erst 27 g Chlorwasserstoff und dann 128 g Schwefeldioxid eingeleitet, wobei sich eine gut rührbare Suspension ausbildet. Unter kräftigem Rühren werden daraufhin bei 10-20 °C innerhalb von 1,5 Stunden kontinuierlich 34 g Methylnitrit zudosiert. Die Methylnitritdosierung ist von einer gleichmäßigen Gasentwicklung im Reaktionsgemisch begleitet. Gegen Ende der Methylnitritdosierung löst sich die Suspension vollständig auf. Die Lösung wird noch 70 Minuten bei 20 °C nachgerührt. Gegen Ende der Reaktion scheidet sich eine geringfügige wässrige Phase ab. Man setzt noch 50 ml Wasser zu, rührt das Gemisch kräftig durch und trennt die wässrige Phase ab. Die organische Phase wird bei 60 °C und 20 mbar eingedampft. Als Rückstand bleiben 124,2 g gelb gefärbtes Öl mit einem über Gaschromatographie bestimmten Gehalt von 92,4 % 2-Trifluormethoxybenzolsulfochlorid zurück. Die Ausbeute beträgt 90,2 % der Theorie.

### Beispiel 5

In einen 1 l Glasreaktor werden 69 g 2-Nitroanilin (98%), 250 ml 1,2-Dichlorethan und eine Lösung von 2 g CuCl₂ x 2H₂O in 3 ml Methanol vorgelegt. In dieses Gemisch werden anschließend bei 0-10°C erst 18 g Chlorwasserstoff und dann 128 g Schwefeldioxid eingeleitet, wobei sich eine gut rührbare Suspension ausbildet. Unter kräftigem Rühren werden daraufhin bei 0-10 °C innerhalb von 1,5 Stunden gleichzeitig 9 g Chlorwasserstoff und 34 g Methylnitrit kontinuierlich zudosiert. Die Methylnitritdosierung ist von einer gleichmäßigen Gasentwicklung im Reaktionsgemisch begleitet. Gegen Ende der Methylnitritdosierung hat sich die Suspension aufgelöst. Die Lösung wird nach vollständiger Methylnitritdosierung noch 2 Stunden bei 5-10 °C nachgerührt. Das Reaktionsgemisch wird mit 50 ml Wasser extrahiert und die organische Phase bei 25 °C und 18 mbar eingedampft. Der Rückstand wird mit 150 ml Wasser verrührt, der gebildete Feststoff über ein Filter abgesaugt, mit 50 ml Wasser gewaschen und getrocknet. Es verbleiben 90,3 g leicht bräunlich gefärbte Kristalle mit einem über HPLC bestimmten Gehalt von 91,8 %. Die Ausbeute beträgt 76,4 % der Theorie.

### Beispiel 6

In einen 0,5 l Glasreaktor werden 64,7 g 3-Amino-2-chlorpyridin (99,3%), 150 ml Tetrahydrofuran und 0,5 g CuCl₂ x 2 H₂O vorgelegt. In dieses Gemisch werden anschließend bei 15-20°C erst 5 g Chlorwasserstoff und dann 128 g Schwefeldioxid eingeleitet, wobei eine fast klare Lösung resultiert. Unter kräftigem Rühren werden daraufhin in das auf -2 bis +2°C temperierte Reaktionsgemisch innerhalb von 4 Stunden gleichzeitig 18 g Chlorwasserstoff und 36 g Methylnitrit kontinuierlich eingeleitet. Die Methylnitritdosierung ist von einer gleichmäßigen Gasentwicklung im Reaktionsgemisch begleitet. Nach vollständiger Methylnitritdosierung wird die Reaktionslösung noch 1,5 Stunden bei ca. 0 °C gerührt.

Im Anschluß daran wird aus dem Reaktionsgemisch der Überschuß an Chlorwasserstoff und Schwefeldioxid sowie ein großer Teil des Lösungsmittels bei 10°C und einem Druck von 30 mbar abdestilliert. Der Rückstand wird in 200 ml Toluol aufgenommen und die Destillation bei 20-25 °C und einem Druck von 35 mbar bis zur weitgehenden Entfernung des Tetrahydrofurans und des bei der Reaktion entstandenen Methanols weitergeführt. Die zurückbleibende toluolische Lösung wird mit 50 ml Wasser extrahiert und die toluolische Phase im Anschluß zur Entfernung des Wassers bei 20 °C und 25 mbar destilliert. Es verbleiben 181,4 g einer gelb-orange gefärbten toluolischen Lösung mit einem Gehalt von 47,7 % 2-Chlor-3-chlorsulfonylpyridin. Die Ausbeute beträgt 81 % der Theorie.

## Patentansprüche

1. Verfahren zur Herstellung von aromatischen oder heteroaromatischen Sulfonsäurechloriden der allgemeinen Formel I 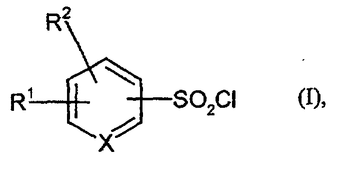 worin
X gleich C oder N,
R¹ gleich Fluor, Chlor, Brom, Nitro, Methoxy, Fluormethoxy, Difluormethoxy, Trifluormethoxy, C₁ - C₄ Alkyl oder Phenyl,
R² gleich H, Fluor, Chlor, Brom, OH, C₁ - C₄ Alkyl sein können,
durch Diazotierung der Verbindungen der allgemeinen Formel II 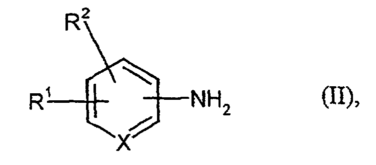 in der
X, R¹ und R² die für die Formel (I) angegebenen Bedeutungen haben,
und Zersetzung der erhaltenen Diazoniumsalze in Gegenwart von Schwefeldioxid und einem Kupferkatalysator, **dadurch gekennzeichnet, daß** die Verbindungen der Formel (II) in einem Gemisch mit einem oder mehreren organischen Lösungsmitteln, Schwefeldioxid und einem Kupferkatalysator bei Temperaturen von -20 bis + 60 °C mit Chlorwasserstoff und Alkylnitrit behandelt werden.

2. Verfahren nach Anspruch 1, **dadurch gekennzeichnet, daß** in den Formeln (I) und (II) R¹ für Fluor, Chlor, Brom, Nitro, Methoxy, Trifluormethoxy, Methyl oder Ethyl und R² für Wasserstoff, Fluor, Chlor, Brom, Methyl oder Ethyl steht.

3. Verfahren nach Anspruch 1, **dadurch gekennzeichnet, daß** als Aniline der Formel (I) 2-Trifluormethoxyanilin, 3-Amino-2-chlorpyridin, 2-Nitroanilin oder 4-Nitroanilin eingesetzt werden.

4. Verfahren nach einem oder mehreren der Ansprüche 1 bis 3, **dadurch gekennzeichnet, daß** als organische Lösungsmittel ein oder mehrere aromatische Kohlenwasserstoffe, halogenierte Kohlenwasserstoffe, Alkohole oder Ether eingesetzt werden.

5. Verfahren nach einem oder mehreren der Ansprüche 1 bis 4, **dadurch gekennzeichnet, daß** Schwefeldioxid in einer Menge von 1 - 8 Moläquivalenten, bevorzugt 1 - 6 Moläquivalenten, besonders bevorzugt 1,5 - 5 Moläquivalenten, bezogen auf die Verbindung der Formel (II) eingesetzt wird.

6. Verfahren nach einem oder mehreren der Ansprüche 1 bis 5, **dadurch gekennzeichnet, daß** als Alkylnitrit Methylnitrit, Ethylnitrit oder Isopropylnitrit eingesetzt wird.

7. Verfahren nach einem oder mehreren der Ansprüche 1 bis 6, **dadurch gekennzeichnet, daß** Chlorwasserstoff in einer Menge von 1 - 4 Äquivalenten, bevorzugt 1 - 2 Äquivalenten, bezogen auf die Verbindung der Formel (II) eingesetzt wird.

8. Verfahren nach einem oder mehreren der Ansprüche 1 bis 7, **dadurch gekennzeichnet, daß** der Kupferkatalysator in einer Menge von 0,001 - 0,5 Mol, bevorzugt 0.005 - 0.1 Mol, pro Mol der Verbindung der Formel (II) eingesetzt.

## Claims

1. Process for the preparation of aromatic or heteroaromatic sulphonyl chlorides of the general formula I 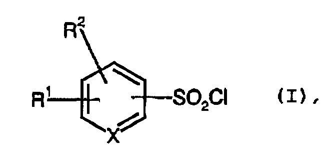 in which
X may be a C or N,
R¹ may be fluorine, chlorine, bromine, nitro, methoxy, fluoromethoxy, difluoromethoxy, trifluoromethoxy, C₁-C₄ alkyl or phenyl,
R² may be H, fluorine, chlorine, bromine, OH, C₁-C₄ alkyl,
by diazotization of the compounds of the general formula II 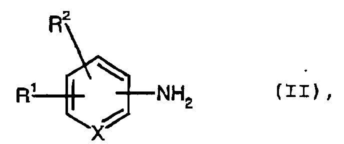 in which
X, R¹ and R² are as defined for formula (I),
and decomposition of the resulting diazonium salts in the presence of sulphur dioxide and a copper catalyst, **characterized in that** the compounds of the formula (II) are treated, in a mixture with one or more organic solvents, sulphur dioxide and a copper catalyst, with hydrogen chloride and alkyl nitrite at temperatures of from -20 to +60°C.

2. Process according to Claim 1, **characterized in that**, in the formulae (I) and (II), R¹ is fluorine, chlorine, bromine, nitro, methoxy, trifluoromethoxy, methyl or ethyl, and R² is hydrogen, fluorine, chlorine, bromine, methyl or ethyl.

3. Process according to Claim 1, **characterized in that** the anilines of the formula (I) used are 2-trifluoromethoxyaniline, 3-amino-2-chloropyridine, 2-nitroaniline or 4-nitroaniline.

4. Process according to one or more of Claims 1 to 3, **characterized in that** the organic solvents used are one or more aromatic hydrocarbons, halogenated hydrocarbons, alcohols or ethers.

5. Process according to one or more of Claims 1 to 4, **characterized in that** sulphur dioxide is used in an amount of 1 - 8 mol equivalents, preferably 1-6 mol equivalents, particularly preferably 1.5 - 5 mol equivalents, based on the compound of the formula (II).

6. Process according to one or more of Claims 1 to 5, **characterized in that** the alkyl nitrite used is methyl nitrite, ethyl nitrite or isopropyl nitrite,

7. Process according to one or more of Claims 1 to 6, **characterized in that** hydrogen chloride is used in an amount of 1 - 4 equivalents, preferably 1 - 2 equivalents, based on the compound of the formula (II).

8. Process according to one or more of Claims 1 to 7, **characterized in that** the copper catalyst is used in an amount of 0.001 - 0.5 mol, preferably 0.005 - 0.1 mol, per mole of the compound of the formula (II).

## Revendications

1. Procédé pour la préparation de chlorures d'acides sulfoniques aromatiques ou hétéroaromatiques de formule générale (I) 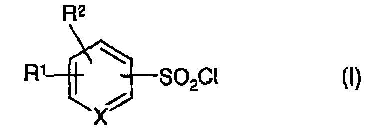 dans laquelle
X représente C ou N,
R¹ représente le fluor, le chlore, le brome, un groupe nitro, méthoxy, fluorométhoxy, difluorométhoxy, trifluorométhoxy, alkyle en C₁-C₄ ou phényle,
R représente H, le fluor, le chlore, le brome, un groupe OH, alkyle en C₁-C₄,
par diazotation de composés de formule générale (II) 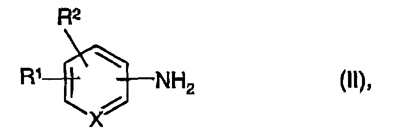 dans laquelle
X, R¹ et R² ont les significations indiquées en référence à la formule (I),
et décomposition des sels de diazonium obtenus en présence de dioxyde de soufre et d'un catalyseur au cuivre, **caractérisé en ce que** l'on traite les composés de formule (II) par le chlorure d'hydrogène et un nitrite d'alkyle à des températures allant de -20 à +60°C dans un mélange d'un ou plusieurs solvants organiques, de dioxyde de soufre et d'un catalyseur au cuivre.

2. Procédé selon la revendication 1, **caractérisé en ce que**, dans les formules (I) et (II), R¹ représente le fluor, le chlore, le brome, un groupe nitro, méthoxy, trifluorométhoxy, méthyle ou éthyle et R² l'hydrogène, le fluor, le chlore, le brome, un groupe méthyle ou éthyle.

3. Procédé selon la revendication 1, **caractérisé en ce que** l'aniline de formule (I) mise en oeuvre est la 2-trifluorométhoxyaniline, la 3-amino-2-chloropyridine, la 2-nitraniline ou la 4-nitraniline.

4. Procédé selon une ou plusieurs des revendications 1 à 3, **caractérisé en ce que** les solvants organiques utilisés consistent en un ou plusieurs hydrocarbures aromatiques, hydrocarbures halogénés, alcools ou éthers.

5. Procédé selon une ou plusieurs des revendications 1 à 4, **caractérisé en ce que** le dioxyde de soufre est mis en oeuvre en quantité de 1 à 8 équivalents molaires, de préférence de 1 à 6 équivalents molaires et dans les meilleures conditions de 1,5 à 5 équivalents molalres par rapport au composé de formule (II).

6. Procédé selon une ou plusieurs des revendications 1 à 5, **caractérisé en ce que** le nitrite d'alkyle mis en oeuvre est le nitrite de méthyle, le nitrite d'éthyle ou le nitrite d'isopropyle.

7. Procédé selon une ou plusieurs des revendications 1 à 6, **caractérisé en ce que** le chlorure d'hydrogène est mis en oeuvre en quantité de 1 à 4 équivalents, de préférence de 1 à 2 équivalents par rapport au composé de formule (II).

8. Procédé selon une ou plusieurs revendications 1 à 7, **caractérisé en ce que** le catalyseur au cuivre est mis en oeuvre en quantité de 0,001 à 0,5 mol, de préférence de 0,005 à 0,1 mol par mole du composé de formule (II).
